(19) Europäisches Patentamt

European Patent Office

Office européen des brevets

(11) **EP 3 846 910 B1**

(12) **EUROPEAN PATENT SPECIFICATION**

(45) Date of publication and mention of the grant of the patent:
**29.10.2025 Bulletin 2025/44**

(21) Application number: **18768837.9**

(22) Date of filing: **07.09.2018**

(51) International Patent Classification (IPC):
**G16H 20/70** (2018.01)   **A61Q 5/10** (2006.01)
**A61K 8/02** (2006.01)   **G06F 17/00** (2019.01)
**G01J 3/46** (2006.01)   **A45D 44/00** (2006.01)

(52) Cooperative Patent Classification (CPC):
**A61Q 5/10; A61K 8/0204; G01J 3/463;**
**G16H 20/70;** A45D 2044/007; G06N 20/00

(86) International application number:
**PCT/EP2018/074083**

(87) International publication number:
**WO 2020/048606 (12.03.2020 Gazette 2020/11)**

(54) **COMPUTER-IMPLEMENTIERTED METHOD OF PRODUCING A RECIPE OF A HAIR COLORING AGENT ASSOCIATED WITH A DESIRED COLORING RESULT**

**COMPUTERGESTÜTZTES VERFAHREN ZUR ERSTELLUNG EINES REZEPTS FÜR EIN HAARFÄRBEMITTEL, DAS MIT EINEM GEWÜNSCHTEN FÄRBEERGEBNIS VERBUNDEN IST**

**PROCÉDÉ INFORMATISÉ DE PRODUCTION D'UNE RECETTE D'AGENT COLORANT POUR CHEVEUX ASSOCIÉE À UN RÉSULTAT DE COLORATION SOUHAITÉ**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.07.2021 Bulletin 2021/28**

(73) Proprietor: **Henkel AG & Co. KGaA**
**40589 Düsseldorf (DE)**

(72) Inventors:
• **BONNIN, Lucile**
**40215 Düsseldorf (DE)**
• **KNUEBEL, Hans Georg**
**40219 Düsseldorf (DE)**
• **KOENEN, Annika**
**41516 Grevenbroich (DE)**

(56) References cited:
**WO-A1-2006/090363   WO-A1-2017/077498**
**WO-A1-96/41139   DE-A1- 102015 225 458**
**DE-A1- 102015 225 459   JP-A- 2007 212 140**
**US-B2- 7 877 294**

• **NITESH V CHAWLA ET AL: "SMOTE: Synthetic Minority Over-sampling Technique", JOURNAL OF ARTIFICIAL INTELLIGENCE RESEARCH, vol. 16, 1 January 2002 (2002-01-01), pages 321 - 357, XP055096179**

EP 3 846 910 B1

## Description

TECHNICAL FIELD

The invention relates to the field of individualized coloration treatments for hair. The invention provides a means to identify which hair coloring agent a user could apply to obtain a coloring result, such as at least a hair color, as close as possible to a desired coloring result.

TECHNOLOGICAL BACKGROUND

For a person who would like to color their hair (have their hair colored), it is important that the achieved coloring result matches the desired hair color.

Hair coloration involves the application of hair coloring agents on hair. Hair coloring agents can comprise a mixture of different dye precursors and can thus also be referred to as a coloring mixture.

While it is possible to calculate the exact color to be achieved and produced in some applied areas of color production, for example with precise photo printing with a calibrated pigment printer, doing so for hair coloration is more complicated.

A reason for this is that when coloring hair, i.e. creating a hair color, it is often the case that dyes are not used, at least not directly, but rather dye precursors. While a coloring process can consist of a multitude of different dyes, it is possible that their colorimetric properties as pure substances are not completely known.

Moreover, concentrations of dyes in the colored hair may be unknown, and it may also be unknown which concentration of the dye in the colored hair corresponds to what concentration of dye precursors in the hair coloring agent. This can be due at least in part to the fact that the combination of dye precursors interacts with each other during the formation of different dyes.

To overcome these constraints, document WO2017/103050 teaches to use predictive analytics on hair data to create a model capable of predicting the color that hair coloring agents of known or unknown composition will have on hair. To build that model, hair data is produced by testing hair coloring agents on different types of hair samples (differing by their initial color, damage state or other state parameters) and measuring the color that can be achieved.

Document WO2017/103056 describes a method using predictive analytics to determine the composition of dyeing agents that could be used to achieve a desired hair color on a type of hair using a coloring process.

The methods described in these documents create a model that can predict the color obtained after applying a hair coloring agent on hair even for hair types or hair coloring agents whose compositions have not been tested in a laboratory. The reliability of the model that is built using predictive analytics increases as more hair color data is added to the algorithm building the model. For underrepresented hair colors, the model might appear to be less accurate.

Most hair coloring agents have compositions which enable to achieve a "natural" hair color which spans shades comprising, mostly "brown" hair colors, black hair color and dark blond hair colors. As a result, there are fewer hair coloring agents available to dye hair into lighter blond, red, orange or other hair color shades. This creates an unequal distribution of hair color data as most measurements provide information relating to slightly different shades of "brown" hair colors. Due to this unequal distribution of existing hair coloring agents, the model that is built using predictive analytics can be less accurate when dealing with hair coloring agents that do not belong to the above-mentioned "brown" hair color shades.

There is therefore a need to provide a more accurate model capable of finding the correspondence between hair coloring agents and desired hair coloring results across a larger spectrum of hair color shades, in particular for hair coloring agents that do not belong to the "brown" hair color shade.

US 7877294 B2 describes a method and system for determining the formula or formulas of haircoloring agents to be used in the process of coloring hair, including the steps of receiving input on the current color, state, and desired color of the hair, and using a database of haircoloring formulas to determine the coloring agents to be used.

WO 2017 077 498 A1 describes a method for computing a haircoloring composition (HCC) or for dispensing ingredients for the HCC are disclosed herein.

WO 2006/090363 A1 describes a system for determining hair color treatment, including a processor.

JP 2007 212140 A describes a prediction method of a hair dyeing color by mixed use of the plurality of hair dyeing agents.

WO 9641139 A1 describes a method and apparatus for determining accurate hair color classifications and appropriate coloring agents.

Chawla, Nitesh & Bowyer, Kevin & Hall, Lawrence & Kegelmeyer, W.. (2002). SMOTE: Synthetic Minority Over-sampling Technique. J. Artif. Intell. Res. (JAIR). 16. 321-357. 10.1613/jair.953 describes an approach to the construction of classifiers from imbalanced datasets.

# EP 3 846 910 B1

## SUMMARY OF THE INVENTION

This disclosure provides a method for producing a recipe of a hair coloring agent with a desired coloring result,

comprising: selecting an initial hair color from a plurality of initial hair colors, selecting the desired coloring result, obtaining hair color data, wherein the hair color data comprises values related to a plurality of hair coloring agents and, for each hair coloring agent from the plurality of hair coloring agents, at least one coloring result,

wherein the at least one coloring result comprises a measured specification about a property of hair color obtained by applying the hair coloring agent to hair,

splitting the hair color data at least into a first hair color group and a second hair color group, the first hair color group comprising more data than the second hair color group, adding artificial hair color data to the second hair color group, wherein the artificial hair color data is generated using the obtained hair color data, and

building a model associating compositions of hair coloring agents to coloring results by employing predictive analytics based on the hair color data and including the artificial hair color data.

The method further comprises:

providing a desired coloring result, and
determining, using the built model, a composition of a hair coloring agent associated with the desired coloring result.

The method further comprises:

determining a recipe of a hair coloring agent associated with the desired coloring result, and
producing the recipe.

The invention improves the accuracy of the model obtained using predictive analytics associating hair coloring agents to achievable hair colors as compared to the methods described in documents WO2017/103056 and WO2017/103050. Hair color data is split into different hair color groups. These groups are created so that they differ by the number of hair coloring agents they comprise. A first hair color group can typically comprise hair coloring agents belonging to the "brown" hair color category. This hair color category could also be labeled as "natural", and further include blond hair color shades. A second hair color group could for example comprise all data available for hair coloring agents belonging to all other hair color categories. These other hair color categories could for example comprise "fashion" colors (red, blue green, orange for example). "Blond" hair color shades could also be put into a separate color category. Additional hair color groups can be defined based on the distribution of hair coloring agents that are available. By defining these hair color groups, the invention can compensate for the lower number of data points in the underrepresented hair color groups by adding artificial data points. These artificial data points can typically correspond to interpolated data points, for which no measurements are available but which are expected to have similar effects on hair color as hair coloring agents for which measured hair color data is available and having close chemical compositions. The artificial data points are created by interpolating existing data points from the second hair color group.

By so doing, the invention improves the accuracy of the model associating hair coloring agents to coloring results that is built using predictive analytics techniques.

A user can easily identify which coloring agent is suitable for a desired coloring result by looking at the correspondence that is made between hair coloring results and hair coloring agents in the model that is built.

The term "hair coloring result" is not to be read in a limitative way encompassing only the color of hair. It can encompass further parameters, for example fastness to washing, hair damage, greyness levels.

Further embodiments are described in the appended claims.

According to an embodiment, the predictive analytics use at least one method from a group of methods, wherein the group of methods includes:

linear or multi-linear regression,
multiple polynomic regression,
a neuronal network method,
a support vector machine method, and
a decision tree method.

According to an embodiment, the hair color data may further comprise for each hair coloring agent from the plurality of hair coloring agents at least one coloring result for different hair types, each hair type being characterized by an initial hair color, wherein the initial hair color is parameterized in a color space.

The color space can for example be a L\*a\*b color space, an RGB color space or any other color space capable of representing colors in a numerical way.

According to an embodiment, hair color data can be added to the second hair color group to obtain a substantially identical number of data points in the first hair color group and in the second hair color group.

Having a substantially identical number of hair coloring agents in all hair color groups reduces the risk of biasing the model that is built using predictive analytics. The term "substantially identical" typically refers to differences in number of hair coloring agents that are below 10%.

According to an embodiment, artificial hair color data can be added to the second hair color group using an oversampling method.

In particular, the oversampling method can be based on a Synthetic Minority Over-Sampling Technique.

According to an embodiment, the hair color data can be split into the first hair color group and the second hair color group based on a color-related feature of the hair coloring agents.

According to an embodiment the hair color data can be split into the first hair color group and the second hair color group based on a semantic analysis of properties of the hair coloring agents in the hair color data.

In particular, hair coloring agents referring to words comprising the term "blond" or synonyms thereof can be put into a second hair color group for "blond" hair coloring agents. Another, third, hair color group could comprise fashionable hair coloring agents whose names comprise words referring to colors such as red, orange, green, purple. The remaining hair coloring agents can be put into the first hair color group for "natural" hair dye colors. These "natural" hair color dyes typically correspond to "brown" or black hair color shades.

According to an embodiment, the hair color data can be split into the first hair color group and the second hair color group based on an assessment of the chemical composition of the hair coloring agents from the plurality of hair coloring agents.

In particular, hair coloring agents comprising pyrazole compounds can be arranged into the second hair color group.

Hair coloring agents comprising pyrazole are known to dye hair in red shades, which are distinct from the common "brown" shades of most hair coloring agents. 1-hydroxyethyl 4,5-Diamino Pyrazole Sulfate is one example of such compounds found in red hair coloring agents.

The method may further combine a splitting into different hair color groups based on chemical compositions with a semantic approach. After separating hair coloring agents comprising pyrazole into a "fashion" hair color group, the invention may use a semantic approach to include further agents into the "fashion" hair color group based on color names or color codes (orange, green, purple) and add further hair color groups for "blond" hair colour shades.

Using the model that is built with predictive analytics, it is possible to determine colors of hair coloring agents that do not exist on the market but which enable to achieve the desired hair coloring result. It is possible to further determine what concentration and mixture of existing hair dye precursors, or what concentration and mixture of existing hair coloring agents is needed to produce such a new hair coloring agent. The invention can further output the recipe or order the production of a new hair coloring agent based on the generated recipe.

According to an embodiment, the method of the invention may further comprise:

outputting a recommendation for a hair coloration product having color properties differing by less than a predetermined threshold from the color properties of the hair coloring agent associated with the desired hair coloring result.

The term "hair coloration product" can refer to a product that is available on the market or a hair coloration composition that is not available on the market but could be produced by mixing dye precursors. Products available on the market can be referred to using reference numbers associated therewith.

The difference between the color properties of the recommended hair coloration product and the color properties of the hair coloring agent determined from the model can be calculated as a distance in a color space, such as the L\*a\*b color space.

According to an embodiment, at least one of the following actions may be further implemented:

outputting an indication of a location where the recommended hair coloration product is available,
requesting an authorization for ordering a sample of the recommended hair coloration product,
ordering the recommended hair coloration product.

The invention also refers to a non-transitory computer readable storage medium having stored thereon a computer program comprising instructions for execution of a method for determination of a correspondence between hair coloring agents and desired hair coloring results as described above.

BRIEF DESRIPTION OF THE DRAWINGS

The present disclosure will hereinafter be described in conjunction with the following drawing figures, wherein like numerals denote like elements, and:

Fig. 1 shows a simplified workflow of the method according to an exemplary embodiment;
Fig. 2 shows schematic representations of non-transitory computer readable storage mediums capable of having stored thereon a computer program intended to implement the method of the invention.

DETAILED DESCRIPTION

The following detailed description is merely exemplary in nature and is not intended to limit the disclosure or the application and uses of the subject-matter as described herein. Furthermore, there is no intention to be bound by any theory presented in the preceding background or the following detailed description.

In various exemplary embodiments, with the determination of the hair coloring agent for the coloring of hair in a desired hair color, desired values for additional properties of hair colors can also be taken into consideration.

In general, properties of hair colors (part of the hair coloring result) can include, for example, hair color information (a color), fastness to washing, light fastness, gray coverage or additional properties. In the process, the hair colors can be created by employing dyeing hair with a hair coloring agent, which is also referred to as a dyeing process.

A "color" can be understood as an interaction of a shade (i.e. a spectral color impression, also referred to as a hue, which can be understood as what is considered the "actual color"), a color intensity (i.e. how intensively the color appears, e.g. compared with a neutral gray tone, which is also referred to as saturation, color saturation, chroma, chromaticity or depth of color) and a brightness (i.e. how light or dark the color appears).

In various exemplary embodiments, the color information can, for example, have a parameterization in a known color space, for example in a L*a*b color space (wherein L* indicates the brightness of a color, a* the portion of green and red and b* the portion of blue and yellow of the color, where the abbreviated form Lab and/or individual L, a and/or b are used here) in an RGB color space with color portions in red, green and blue, in a CMYK color space with color portions in cyan, magenta, yellow and black or in any other arbitrary color space. The term "shade" can be understood, as described above, to mean the spectral color impression of a color independently of how it can be parameterized, such as a point in a two-dimensional color space (e.g. a*b* of the L*a*b* system) or a ratio of color portions (such as in the RGB color space or in the CMYK color space) .

In various exemplary embodiments, a color space from which the color information (e.g. the hair color information of the colored hair or the hair before the coloring, which is also referred to as the initial hair color) arose, or in which the color information is represented (for example, if a hair color is represented, see below) can be procured so that a determined or represented color is independent of a medium through which the color is determined or represented (e.g. color measuring device, screen, printer, scanner, human eye, etc.). The color space can be, for example, an L*a*b* color space and the color information can, for example, be a shade parameterized by employing a* and b*. The uniform representation in the medium-independent color space can make it possible, for example, to present a close-to-reality coloring result to be expected, for example, in which the same color impression of a color achieved by coloring is left on the observer in a representation of the result to be expected, for example as printing on a package, an advertisement on a computer screen, etc.

Hair coloring agents can have a mixture of different dye precursors and/or dyes and can thus also be referred to as a coloring mixture.

Table 1 shows a portion of hair color data listing come known hair coloring agents identified by their reference name and their color as expressed in the L*a*b color space.

| Hair coloring agent (reference) | L*a*b color | Category |
|---|---|---|
| N&E 542 medium ash blond | 40,4 3,8 11,5 | blond |
| N&E 545 medium gold blond | 38,7 7,3 12,7 | blond |
| N&E 550 dark blond | 42,7 4,0 15,2 | blond |
| N&E 555 dark blond | 38,5 7,6 13,0 | blond |
| N&E 557 multi-reflex brown | 31,7 8,9 13,7 | brown (natural) |
| N&E 550 light brown | 26,5 3,5 7,6 | brown (natural) |
| N&E 562 light ash brown | 29,5 3,2 7,4 | brown (natural) |
| M&E 555 light gold brown | 33,4 5,5 11,4 | brown (natural) |
| N&E 566 cinnamon golden brown | 25,2 6,7 10,0 | brown (natural) |

(continued)

| Hair coloring agent (reference) | L*a*b color | Category |
|---|---|---|
| *N&E 568 intense red* | *31,9 30,8 20,6* | *Red (fashion)* |
| N&5 570 medium brown | 27,0 3,7 9,6 | brown (natural) |
| N&E 574 dark chocolate | 25,9 3,0 10,1 | brown (natural) |
| N&E 575 chestnut red brown | 25,5 8,3 7,7 | brown (natural) |
| N&E 580 dark brown | 20,8 2,1 4,5 | brown (natural) |
| N&E 584 mocha chocolate | 23,7 6,5 7,3 | brown (natural) |
| N&E 585 multi-reflex brown | 23,3 8,3 5,2 | brown (natural) |
| N&E 586 cinnamon dark brown | 23,2 7,1 8,7 | brown (natural) |
| *N&E 588 glossy acaiberry* | *22,9 15,5 2,9* | *red (fashion)* |
| N&E 590 black | 15,3 0,1 -1,7 | black (natural) |
| *Nectra 688* | *25,6 10,4 6,6* | *red (fashion)* |
| *Nectra 499* | *20,7 11,6 -2,0* | *purple (fashion)* |
| Nectra 568 | 29, 6 10,4 6,6 | brown (natural) |
| Nectra 400 | 21,9 1,8 2,7 | brown (natural) |
| Syoss Oleo 3-10 | 20,3 2,3 2,7 | brown (natural) |
| *Syoss Oleo 5-92* | *30,5 31,7 13,2* | *red (fashion)* |
| Nectra 320 | 19,2 1,3 1,9 | brown (natural) |
| Nectra 468 | 29,2 11,5 10,5 | brown (natural) |
| *Cashmere red variant 2* | *31,4 36,1 22,6* | *red (fashion)* |
| *Syoss Color 2014 5-22* | *25,3 25,3 12,5* | *red (fashion)* |
| Syoss Color 2014 1-4 | 15,0 12 -3,3 | brown (natural) |
| Syoss Color 2014 5-28 | 23,2 9,1 9,6 | brown (natural) |
| *Syoss Oleo 4-29* | *25,7 26,0 13,7* | *red (fashion)* |
| Syoss Oleo 1-40 | 15,3 1,4 -3,3 | brown (natural) |
| *Igora Royal 5-88* | *25,1 26,5 12,6* | *red (fashion)* |
| Nectra 600 | 29,8 4,6 10,0 | brown (natural) |
| Nectra 662 | 29,3 6,7 9,4 | brown (natural) |
| *Nectra 777* | *45,3 29,7 33,2* | *orange (fashion)* |
| Igora Royal 3-0 | 18,3 1,1 1,8 | brown (natural) |
| Igora Royal 5-5 | 31,0 6,7 11,3 | brown (natural) |
| Igora Royal 6-65 | 33,0 7,1 11,8 | brown (natural) |
| Igora Royal 6-0 | 28,8 4,1 8,5 | brown (natural) |
| Igora Royal 5-1 | 28,3 1,4 5,2 | brown (natural) |
| *Igora Royal 4-88* | *25,1 26,5 14,4* | *purple (fashion)* |
| *Igora Royal 7-887* | *35,1 40,7 29,0* | *red (fashion)* |
| *Syoss Color 2012 4-2* | *25,5 24,2 13,3* | *red (fashion)* |
| *Syoss Color 2012 5-22* | *30,3 31,6 19,3* | *red (fashion)* |
| Syoss Color 2012 1-4 | 16,8 1,7 -5,4 | black (natural) |
| *Syoss Color 2D12 5-29* | *33,5 36,1 22,7* | *red (fashion)* |

(continued)

| Hair coloring agent (reference) | L*a*b color | Category |
|---|---|---|
| Syoss Color 2012 3-55 | 27,7 6,9 9,3 | brown (natural) |
| Syoss Color 2012 5-0 | 25, 6 3,0 6, 6 | brown (natural) |
| Brilliance 880 | 25,2 25 6,3 | brown (natural) |
| Igora Royal 1-0 | 16,8 0,1 -1,6 | black (natural) |
| Syoss Oleo 2-10 | 15,7 1,1 0,3 | brown (natural) |
| Syoss Oleo 4-18 | 24,9 6,3 8,2 | brown (natural) |
| **Syoss Oleo 6-10** | **27,4 46 10,6** | **blond** |

As can be seen on this table "brown" hair coloring agents (displayed with no emphasis in table 1 above) represent the majority of the colors available on this list. Only 5 out of 55 of the listed products correspond to "blond" hair coloring agents (indicated in bold) and 15 out of 55 to other hair coloring agents (in particular red hair coloring agents, underlined and in italic).

The hair color data further comprises measurements corresponding to the color of hair resulting from application of the hair coloring agent on hair. Once these measurements are incorporated in the hair color data, hair color data is split into at least two hair color groups. A first hair color group comprises hair colors that are overrepresented, typically in the "brown" or "black" hair color shades. This creates a hair color group that can be named "natural". Darker blond shades can also be associated to this hair color category. A second hair color group typically comprises other colors of hair coloring agents, for example light blond shades or other "fashion" color shades, such as red, blue, green, orange colors.

To separate hair data into these hair color groups, it is possible to use a semantic approach identifying key words in the reference of the hair coloring agents. For example, hair coloring agents whose references comprise the word "brown" or synonyms thereof could be classified into the first hair color group. Hair coloring agents whose references comprise the word "blond" or equivalent terms such as for example "platinum", could be classified into a second hair color group.

Further hair color groups can be created to ensure a more accurate model is built for predicting hair coloring agents needed to achieve a desired hair color using predictive analytics. For example, it is possible to have a third hair color group for "fashion" colors. This third hair color group can include references that comprise words referring to other colors such as "red", "cherry", "copper", "violet", "orange", "blue" for example.

In addition to a semantic splitting of hair color data, it is possible to rely on naming rules used to refer to hair coloring agents. Some hair coloring agents might be named according to a code which could also be identified to sort hair color data into different hair color groups.

Another possible way of splitting data into different hair color groups can consist in using the measured color of the hair coloring agent represented in the L*a*b color space or any other color space such as the RGB color space. Hair color data comprising hair coloring agents which have similar numerical values in the color space in which they are expressed can be clustered into a same hair color group. When the distance between two colors that are closest neighbors in the color space is above a predetermined threshold, these colors can be considered as belonging to different hair color groups.

When the chemical composition of the hair coloring agent is accessible, hair color data can be split into different hair color groups based on the presence of particular chemical compounds. For example, pyrazole based compounds are generally used to create red hair coloration precursors. The presence of such components and in particular the presence of 1-hydroxyethyl 4,5-Diamino Pyrazole Sulfate could be used to put hair color data into a second hair color group for example.

Once hair data is split into at least a first and a second hair color groups, the hair color groups that comprise fewer data points than the first hair color group (associated to common hair coloring agents, typically "brown" hair color shades) are enhanced by adding artificial hair color data thereto.

To add these artificial hair color data different interpolation methods could be used. One example is to change the color of an existing hair coloring agent by a few percent in the hair color space (L*a*b or RGB for example). Hair coloring parameters for different hair types can be assigned to this artificial hair coloring agent by calculating an average value of each hair coloring parameter using values available for closest neighbors of that artificial hair coloring agent in the color space.

The technique used to add artificial hair color data by introducing new hair coloring agents can be chosen among oversampling techniques.

For example, a Synthetic Minority Oversampling technique (also known as "SMOTE") can be used. "SMOTE" is an oversampling technique that is for example used to add virtual data in imbalanced data sets in the technical fields of telecommunication management, fraudulent telephone calls, text classification or detection of oil spills from satellite images. A detailed description of this technique can be found in the article by Nitesh V. Chawla et al. "SMOTE: Synthetic Minority Over-sampling Technique) published in the Journal of Artificial Intelligence Research 16 (2002) 321-357.

As explained in this article, the minority class (second and further hair color groups) is over-sampled by taking each minority class sample (data point among the hair color data) and introducing artificial data points along the line segments joining any/all of the k minority class nearest neighbors (k being an integer advantageously selected to be equal to 5 for hair coloring applications in this embodiment). Depending upon the amount of over-sampling required, neighboring data points from the k nearest neighbors are randomly chosen. For instance, if the amount of over-sampling needed is 200% (which multiplies the total number of data points in the hair color group by a factor of three), only two neighbors from the five nearest neighbors are chosen and one sample is generated in the direction of each of these two neighbors. Artificial data points are generated in the following way: the difference between the feature vector (value associated to a data point) under consideration and its nearest neighbor is calculated. This difference is multiplied by a random number between 0 and 1 and is added to the feature vector under consideration. This causes the selection of a random point along the line segment between two specific features.

By adding artificial data points to hair color groups corresponding to hair colors other than the "natural" or "brown" hair color shades, the invention improves the accuracy of the model that is built using predictive analytics. The artificial data points give a substantially identical statistical weight to hair color data in all hair color groups, which forces the model that is built using predictive analytics to treat all hair color groups substantially equally and to build a model that is equally reliable in underrepresented hair color groups and in the "brown" or "natural" hair color group.

Applicant has noticed that in the context of oversampling hair coloring agents, it is advantageous to oversample the hair data by a factor of 1,5. In other words, 150% artificial hair data points are introduced in the hair color groups that differ from the "brown" hair color shades group. This parameter can be adjusted as the relative proportion of available hair coloring agents changes over time, for example when some hair colors become more popular and offer more diversity in the choice of hair coloring agents to dye hairs in such color shades.

To determine expected hair colors for a (e.g. arbitrary) initial state and for a large number of combinations of relevant concentrations of a plurality of dye precursors, methods from the field of predictive analytics (also known as "big data", "data mining" or "machine learning") can be applied, using the hair color data including the added artificial hair color data. Other parameters of the dyed hair can also be predicted such as fastness to washing, gray coverage and/or light fastness, despite the many parameters that they introduce in the model that is built. Two examples of such a predictive analytics approach are described in documents WO2017/103056 and WO2017/103050.

The large number of combinations of concentrations of the plurality of dye precursors can have, for example, about $10^5$ combinations or more, or, for example, a continuous distribution of values for the concentrations of the plurality of dye precursors. Only a small portion of these combinations are available for use (via for example a product on the market) in the form of hair coloration agents. Measurements are made on these hair coloration agents, by testing the coloration they produce when applied to different types of hair, to produce hair color data.

The word "types of hair" typically refers to the initial hair color, but can further also comprise parameters of the initial hair such as greyness or hair damage level for example.

A relationship between a hair coloring agent, a hair type to which the hair coloring agent is applied and at least one coloring result can be determined by employing predictive analytics using hair color data.

It is now possible to guarantee a color result to a user (e.g. a consumer) that is as close as possible to their desired hair color (insofar as it is chemically possible) by employing the methods from predictive analytics, on both the available hair color data and the artificial hair color data that was added to some of the hair color groups as explained above. Predictive analytics thus builds a more accurate model correlating hair coloring agents to the hair color that can be achieved when the hair coloring agent (represented for example by its color in the L*a*b, RGB or CMYK color space) is applied to a hair type. Furthermore, after such a model has been built, the model can be proof-tested using for example the "leave one out" cross-validation method. This cross-validation method consists in taking a data point out of the hair color data and to check whether the values predicted by the model for this data point match the values obtained experimentally.

Additional examples and embodiments on how the predictive analytics method uses hair data to construct a model can be found in documents WO2017/103056 and WO2017/103050.

A value for an initial color of the hair to be colored can be determined. The initial hair color can be parameterized in a color space, such as a L*a*b* color space, an RGB color space or a CMYK color space for example.

In various exemplary embodiments, an initial hair color can be selected from a plurality of initial hair colors, for example by a hair styling professional or by the person to whom the hair belongs. The initial hair color can for example be chosen as being the one that makes the greatest color impression on a person observing the hair. The determination of the initial hair color can also be done using a device or a data processing of a picture of the hair of a user.

In various exemplary embodiments, a plurality of hair colors achievable by coloring with an available hair coloring agent can be determined from a plurality of available hair coloring agents.

In determining the achievable hair colors in various exemplary embodiments, at least one desired value for an additional (e.g. in addition to the hair color) coloring result can be taken into consideration. For example, when determining the achievable hair colors, the achievable hair colors are limited to those which also have the (e.g. minimum) value for the at least one additional coloring result, such as a required (minimum) fastness to washing or a required (minimum) gray coverage.

In various exemplary embodiments, the plurality of available hair coloring agents can have a plurality of pre-packaged hair coloring agents, such as hair coloring agents which are commercially available. In various exemplary embodiments, the plurality of available hair coloring agents can include a plurality of hair coloring agents having a previously unknown, unused or uncolored combination of concentrations of dye precursors. In various exemplary embodiments, the plurality of available hair coloring agents can have both prepackaged and unknown hair coloring agents.

In various exemplary embodiments, the method can be executed by employing a data processing device.

The data processing device can, for example, be a computer or any other data processing device which is suitable to store and prepare data and execute the predictive analytics, i.e. any data processing device with an adequately large data memory and sufficiently powerful processor.

The selection of the desired hair color can take place, for example, by selection according to a presentation of achievable hair colors in various exemplary embodiments. The achievable hair colors can, for example, be presented by employing a display device, e.g. a screen, e.g. a computer screen, or by employing another output device, such as a printer. With presentation, the achievable hair colors can be presented, for example, as colors. In the process, the hair colors can be parameterized, for example, in a medium-independent color space, e.g. in the L*a*b* color space, which enables a realistic representation of the achievable hair colors, or the hair colors can be transformed from a color space to a color space which is expected by the output device. In various exemplary embodiments, the reproduction of the colors themselves can be omitted. Instead, values for a respective parameterization in the color space, for example, can be specified.

In various exemplary embodiments, the selection can include input of the selection into a data processing device, i.e. a computer. In the process, the input device can have any type of input, such as touching a screen, clicking in a screen area with a mouse, entering information by employing a keyboard and a voice command.

In various exemplary embodiments, specification of a desired coloring result in the form of a desired hair color can take place before the presentation of the achievable hair colors or even before determination of the achievable hair colors, for example, on the basis of their values for the parameterization in the color space or a presentation of such generally parameterized colors.

In such cases, selection of the desired hair color can take place automatically. Insofar as the predetermined desired hair color is one of the achievable hair colors, it can be selected automatically as a desired hair color.

If the actual desired hair color is not an achievable hair color, the presentation of the achievable hair colors comprises specification by the color difference ($\Delta E$) from the actual desired hair color.

In various exemplary embodiments, there can be a plurality of hair coloring agents which result in the desired hair color (with or without defined tolerance range) by coloring the hair. In such cases, the hair coloring agent can be selected arbitrarily, or additional criteria can be used to determine the hair coloring agent, such as a price for the hair coloring agent, an effect on the hair (e.g. whether and, if applicable, how heavily the hair coloring agent can damage the hair), an availability of the hair coloring agent, etc.

In various exemplary embodiments, with use of the selected desired hair color, a color difference between a plurality of pre-packaged hair coloring agents (which can also be referred to as retail products) and the desired hair color can be determined.

In various exemplary embodiments, the hair coloring agent having the smallest color difference from the desired hair color according to the correspondence determined in consideration of the initial state parameters can be selected from the pre-packaged hair coloring agents as the hair coloring agent for coloring hair in the desired hair color.

Alternatively, the color difference can be weighted in various exemplary embodiments. If, for example, the exact hue (H) of the desired hair color should be more important than the exact lightness (L), $\Delta H$ should have a greater influence on the color difference $\Delta E$ than $\Delta L$.

In various exemplary embodiments, the hair coloring agent having a smallest hue difference $\Delta H$, lightness difference $\Delta L$ or chroma difference $\Delta C$ from the desired hair color according to the relationship determined in consideration of the initial state parameters can be selected from the pre-packaged hair coloring agents as the hair coloring agent for coloring hair in the desired hair color.

In various embodiments, when, for example, additional or alternative hair coloring agents to the pre-packaged hair coloring agents cannot also be used, the desired hair color can, descriptively expressed, be plugged into the relationship (the model) as an initial parameter in order to obtain an assigned coloring agent which produces the desired hair color with the coloring pre-condition parameters, such as a combination of coloring agent precursors.

In various exemplary embodiments, the measured specifications of the hair color data, the properties of the hair color generated by coloring can be described (e.g. L*, a*, b* for the color, a fastness to washing, light fastness, gray coverage, etc.) by independent variables. The dependency of the dependent variables on independent variables (such as the concentrations of dye precursors, such as the concentrations as they are applied on the head ("on head")) can be modeled by employing complex mathematical models, which can be found by employing the predictive analytics method. This means a relationship between the independent and the dependent variables (in other words, between the parameters of hair coloring agents, hair types and the coloring results) can be determined by employing the predictive analytics method. For example, this can be expressed as:

$$L*a*b* = f(c_1, c_2, c_3, ..., c_n),$$

wherein L*a*b denotes the color parameters, $c_n$ (n=1,...,n, n>1) denotes concentrations of dye precursors. In the process, the function can be known analytically or not. If no analytical function is known, the values of the dependent variables (of the coloring results) are also calculated by employing numerical algorithms.

In addition to dye precursors, dyes can also be used in hair coloring agents. Accordingly, $c_i$ can also denote concentrations of dyes.

Possible independent variables can be metric (cardinal), ordinal or categorial.

In various exemplary embodiments, the independent variables (the coloring pre-condition parameters) can be properties which influence the coloring result, for example the concentration of a respective dye precursor, the base hair color, damage condition and/or a degree of graying of the hair or similar.

Predictive analytics can be generally described as a method for extracting information from large amounts of data and generating a model from said data which make it possible to also make predictions for values that are not part of the data set. Using a predictive analytics method, part of the data set can be typically used as a training data set (also referred to as a training set or training data). Based on this training data set, one or multiple models can be generated, which can be tested on the basis of data which is not part of the training data set, on the basis of the overall data, or on the basis of a specially selected part of the data.

For example, a determination measure $R^2$, a mean absolute error, a mean quadratic error, a standard deviation and/or a mean deviation can be used for evaluation of the model, i.e. determination of the adaptation quality.

The determination measure $R^2$ can correspond to a squared correlation coefficient for a linear regression model. It can be defined differently for a different model (a different relationship).

Various functions or methods can be used for modeling by employing predictive analytics according to different exemplary embodiments. In a simple case, for example, a multiple linear regression can be used. Better results can be typically achieved using multiple polynomic regressions, neuronal networks, support vector machines, decision trees (e.g. tree ensembles) or similar methods.

In various exemplary embodiments, a method for computer-aided determination of a hair coloring agent for the coloring of hair in a desired hair color can be based on the following sequence (a so-called workflow):

1. determination of an initial hair color of a user (e.g. a consumer), e.g. on different portions of their hair (e.g. the hairstyle), e.g. base, tips, roots or lengths of the hair, particularly on a portion of the hair estimated to be representative by the user;

2. determination (e.g. classification) of additional relevant initial state parameters, e.g. hair damage (degree of damage) and/or a degree of graying;

3. determination of a desired result (desired hair color) of the user, such as a combination of values in the L*a*b* color space;

4. calculation of a plurality of hair colors which are achievable, for example, on hair portions estimated to be representative by employing all available pre-packaged coloring agents (e.g. available in retail or from a hairdresser (retail products));

5. determination (e.g. calculation) of a color difference $\Delta E$ for all combinations of achievable hair color and desired hair color; and

6. selection of the pre-packaged coloring agent with the smallest color difference $\Delta E$.

In various alternative exemplary embodiments, a method for computer-supported determination of a hair coloring agent for the coloring of hair in a desired hair color can be limited for the hair coloring agent to include an existing portfolio of (prepackaged) hair coloring agents (and/or their recipes) as well as all feasible recipes in a specified n-dimensional dye space (with n dye precursors) with comprehensive permutation of all theoretical dye precursor combinations and to calculate a color result (e.g. a plurality of achievable hair colors) on an initial position (e.g. a plurality of initial parameters) of the user. The method can include a sequence according to the following:

1. determination of an initial hair color of a user (e.g. a consumer), e.g. on different portions of their hair (e.g. the hairstyle), e.g. base and tips of the hair, particularly on a portion of the hair estimated to be representative by the user;

2. determination (e.g. classification) of additional relevant initial state parameters, e.g. hair damage (degree of damage) and/or a degree of graying;

3. determination of a desired result (desired hair color) of the user, such as a combination of values in the L*a*b* color space;

4. Identification of highly-relevant and less-relevant dyes (e.g. dye precursors) in a predictive analytics model (this can also be referred to as "feature selection");

5. simulation of a large number (e.g. >> about $10^5$) of theoretical recipes on the representative hair color (a base hair color which is a hair portion estimated to be representative) of the user;

6. determination (e.g. calculation) of a color difference $\Delta E$ for all combinations of achievable hair color and desired hair color; and

7. selection of the theoretical recipe with the smallest color difference $\Delta E$ as the hair coloring agent;

8. production according to this exact recipe, such as on special production lines or directly at the place of purchase (also referred to as point of sale).

Alternatively to the color state $\Delta E$, the hue difference $\Delta H$, the lightness difference $\Delta L$ or the chroma difference $\Delta C$ can also be taken into consideration in the method.

In various exemplary embodiments, a data processing device for execution of a computer-aided determination of a hair coloring agent for coloring of hair in a desired hair color is prepared, wherein the data processing device can be set up to execute the method described herein.

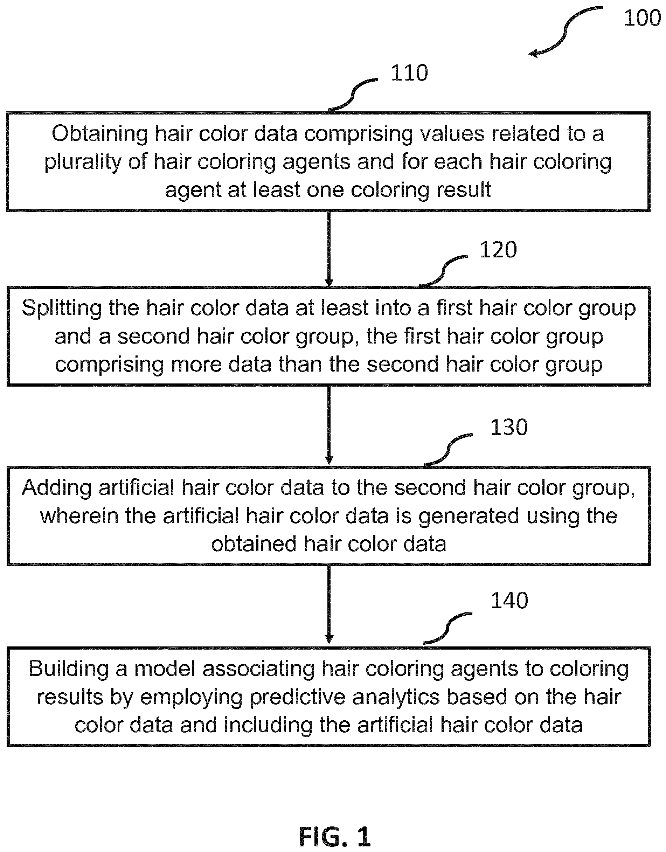

Figure 1 provides a basic workflow of the method 100 used by the invention to build a model of associating hair coloring agents to hair coloring results.

At first, the method involves obtaining 110 hair color data, these hair color data comprising hair coloring agents and at least one hair coloring result for each hair coloring agent. More than one hair coloring result is possible for one hair coloring agent, provided further information regarding an initial state of hair, such as for example damage state, initial hair color, greyness of hair, is available.

Then, the method comprises a splitting 120 of the hair color data into at least a first hair color group and a second hair color group, as explained above. In that process, the second hair color group and all further possible hair color groups comprise a different number of data points that the first hair color group.

The method further proceeds by adding 130 artificial hair color data using the obtained hair color data. An interpolation method such as the SMOTE technique described above can be used for that purpose.

Finally, the method proceeds by building 140 a model associating hair coloring agents to hair coloring results by employing predictive analytics on the obtained hair color data and the artificial hair color data.

After establishing this model, the invention may further comprise a step of recommending a hair coloration product which represents a closest color match between a desired hair coloration result input by the user and a predicted hair coloration result achievable with existing or manufacturable hair coloration agents.

An ordering of such hair coloration agents or hair coloration products could be implemented automatically, after confirmation received to a request sent to a user or via a command (for example typed or dictated) received from the user.

The method may also output a location where the recommended hair coloration product or hair coloring agent is available.

The steps of the examples and embodiments described above can be implemented by a processor such as a computer. A computer program product comprising steps of the above-described method can be used to implement the method on a computer.

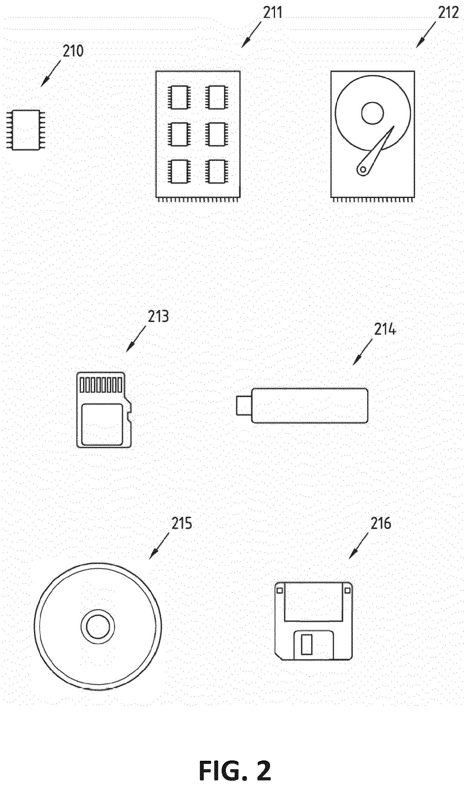

Figure 2 provides examples of non-transitory computer readable storage mediums on which a computer program comprising instructions to implement the method of the invention can be stored. These could for example comprise a processor or chip 210, an electronic circuit comprising several processors or chips 211, a hard drive 212, a flash or SD card 213, a USB stick 214, a CD-ROM or DVD-ROM or Blue-Ray disc 215, or a diskette 216.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the various embodiments in any way. Rather, the foregoing detailed description will provide those skilled in the art with a convenient road map for implementing an exemplary embodiment as contemplated herein. It being understood that various changes may be made in the function and arrangement of elements described in an exemplary embodiment without departing from the scope of the various embodiments as set forth in the appended claims.

**Claims**

1. A method (100) implemented by a processor for producing a recipe of a hair coloring agent associated with a desired coloring result, comprising:

selecting an initial hair color from a plurality of initial hair colors,
selecting the desired coloring result,
obtaining (110) hair color data, wherein the hair color data comprises values related to a plurality of hair coloring agents and, for each hair coloring agent from the plurality of hair coloring agents, at least one coloring result, wherein the hair color data further comprises for each hair coloring agent from the plurality of hair coloring agents at least one coloring result for different hair types, each hair type being associated with an initial hair color, wherein the at least one coloring result comprises a measured specification about a property of hair color obtained by applying the hair coloring agent to hair,
splitting (120) the hair color data at least into a first hair color group and a second hair color group, the first hair color group comprising more data than the second hair color group,
adding (130) artificial hair color data to the second hair color group, wherein the artificial hair color data is generated using the obtained hair color data, wherein artificial hair color data is added to the second hair color group using an oversampling method, and
building (140) a model associating hair coloring agents to coloring results by employing predictive analytics based on the hair color data and including the artificial hair color data,
determining, using the built model, the hair coloring agent associated with the desired coloring result, wherein the hair coloring agent is a hair coloration product which represents a closest color match between the desired coloring result and hair coloring results achievable with existing or manufacturable hair coloration agents,
determining the recipe of the hair coloring agent associated with the desired coloring result, and
producing the recipe.

2. The method according to claim 1, wherein the predictive analytics use at least one method from a group of methods, wherein the group of methods includes:

linear or multi-linear regression,
multiple polynomic regression,
a neuronal network method,
a support vector machine method, and
a decision tree method.

3. The method according to any one of the previous claims, wherein the initial hair color is parameterized in a color space.

4. The method according to any one of the preceding claims, wherein artificial hair color data is added to the second hair color group to obtain a substantially identical number of data points in the first hair color group and in the second hair color group.

5. The method according to claim 1, wherein the oversampling method is based on a Synthetic Minority Over-Sampling Technique.

6. The method according to any one of the preceding claims, wherein the hair color data is split into the first hair color group and the second hair color group based on a color-related feature of the hair coloring agents.

7. The method according to any one of the preceding claims, wherein the hair color data is split into the first hair color group and the second hair color group based on a semantic analysis of properties of the hair coloring agents in the hair color data.

8. The method according to any one of the preceding claims, wherein the hair color data is split into the first hair color group and the second hair color group based on an assessment of the chemical composition of the hair coloring agents from the plurality of hair coloring agents.

9. The method according to claim 8, wherein hair coloring agents comprising pyrazole compounds are arranged into the second hair color group.

10. A non-transitory computer readable storage medium (210-216) having stored thereon a computer program comprising instructions for execution of a method for producing a recipe of a hair coloring agent associated with a desired coloring result according to any one of the preceding claims.

**Patentansprüche**

1. Ein Verfahren (100), das von einem Prozessor implementiert wird, um ein Rezept für ein Haarfärbemittel zu erstellen, das mit einem gewünschten Färbeergebnis verbunden ist, umfassend:

   Auswählen einer Ausgangsfarbe aus einer Vielzahl von Ausgangsfarben,
   Auswählen des gewünschten Färbeergebnisses,
   Erhalten (110) von Haarfarbdaten, wobei die Haarfarbdaten Werte umfassen, die sich auf eine Vielzahl von Haarfärbemitteln beziehen, und für jedes Haarfärbemittel aus der Vielzahl von Haarfärbemitteln mindestens ein Färbeergebnis,
   wobei die Haarfarbdaten ferner für jedes Haarfärbemittel aus der Vielzahl von Haarfärbemitteln mindestens ein Färbeergebnis für verschiedene Haartypen umfassen, wobei jeder Haartyp mit einer Ausgangshaarfarbe in Verbindung steht,
   wobei das mindestens eine Färbeergebnis eine gemessene Spezifikation über eine Eigenschaft der Haarfarbe umfasst, die durch Auftragen des Haarfärbemittels auf das Haar erhalten wurde,
   Aufteilen (120) der Haarfarbdaten in mindestens eine erste Haarfarbgruppe und eine zweite Haarfarbgruppe, wobei die erste Haarfarbgruppe mehr Daten umfasst als die zweite Haarfarbgruppe,
   Hinzufügen (130) künstlicher Haarfarben-Daten zur zweiten Haarfarben-Gruppe, wobei die künstlichen Haarfarben-Daten unter Verwendung der erhaltenen Haarfarben-Daten erzeugt werden, wobei künstliche Haarfarben-Daten unter Verwendung eines Oversampling-Verfahrens zur zweiten Haarfarben-Gruppe hinzugefügt werden, und
   Erstellen (140) eines Modells, das Haarfärbemittel mit Färbeergebnissen verknüpft, indem eine prädiktive Analyse auf der Grundlage der Haarfarbdaten unter Einbeziehung der künstlichen Haarfarbdaten durchgeführt wird,
   Bestimmen des Haarfärbemittels, das mit dem gewünschten Färbeergebnis assoziiert ist, unter Verwendung des erstellten Modells, wobei das Haarfärbemittel ein Haarfärbeprodukt ist, das die beste Farbübereinstimmung zwischen dem gewünschten Färbeergebnis und den mit vorhandenen oder herstellbaren Haarfärbemitteln erzielbaren Haarfärbeergebnissen darstellt,
   Bestimmen der Rezeptur des Haarfärbemittels, das mit dem gewünschten Färbeergebnis assoziiert ist, und
   Herstellen der Rezeptur.

2. Verfahren nach Anspruch 1, wobei die prädiktive Analyse mindestens ein Verfahren aus einer Gruppe von Verfahren verwendet, wobei die Gruppe von Verfahren umfasst:

   lineare oder multilineare Regression,
   multiple polynomische Regression,
   ein neuronales Netzwerkverfahren,
   eine Support-Vektor-Maschinen-Methode und
   eine Entscheidungsbaummethode.

3. Verfahren nach einem der vorstehenden Ansprüche, wobei die ursprüngliche Haarfarbe in einem Farbraum parametrisiert wird.

4. Verfahren nach einem der vorstehenden Ansprüche, wobei künstliche Haarfarbdaten zur zweiten Haarfarbgruppe hinzugefügt werden, um eine im Wesentlichen identische Anzahl von Datenpunkten in der ersten Haarfarbgruppe und in der zweiten Haarfarbgruppe zu erhalten.

5. Verfahren nach Anspruch 1, wobei das Oversampling-Verfahren auf einer synthetischen Minority-Oversampling-Technik basiert.

6. Verfahren nach einem der vorstehenden Ansprüche, wobei die Haarfarbdaten auf der Grundlage eines farbbezogenen Merkmals der Haarfärbemittel in die erste Haarfarbgruppe und die zweite Haarfarbgruppe aufgeteilt werden.

7. Verfahren nach einem der vorstehenden Ansprüche, wobei die Haarfarben-Daten auf der Grundlage einer semantischen Analyse der Eigenschaften der Haarfärbemittel in den Haarfarben-Daten in die erste Haarfarbengruppe und die zweite Haarfarbengruppe aufgeteilt werden.

8. Verfahren nach einem der vorstehenden Ansprüche, wobei die Haarfarbdaten auf der Grundlage einer Bewertung der chemischen Zusammensetzung der Haarfärbemittel aus der Vielzahl von Haarfärbemitteln in die erste Haarfarbgruppe und die zweite Haarfarbgruppe unterteilt werden.

9. Verfahren nach Anspruch 8, wobei Haarfärbemittel, die Pyrazolverbindungen enthalten, in der zweiten Haarfarbengruppe angeordnet sind.

10. Nicht-transitorisches, computerlesbares Speichermedium (210-216), auf dem ein Computerprogramm gespeichert ist, das Anweisungen zur Ausführung eines Verfahrens zur Erstellung einer Rezeptur für ein Haarfärbemittel umfasst, das mit einem gewünschten Färbeergebnis gemäß einem der vorstehenden Ansprüche verbunden ist.

**Revendications**

1. Procédé (100) mis en œuvre par un processeur pour produire une recette d'un agent colorant capillaire associé à un résultat de coloration souhaité, comprenant :

la sélection d'une couleur de cheveux initiale parmi une pluralité de couleurs de cheveux initiales,
sélectionner le résultat de coloration souhaité,
l'obtention (110) de données de couleur de cheveux, dans lequel les données de couleur de cheveux comprennent des valeurs liées à une pluralité d'agents colorants pour cheveux et, pour chaque agent colorant pour cheveux parmi la pluralité d'agents colorants pour cheveux, au moins un résultat de coloration,
dans lequel les données de couleur de cheveux comprennent en outre, pour chaque agent colorant capillaire parmi la pluralité d'agents colorants capillaires, au moins un résultat de coloration pour différents types de cheveux, chaque type de cheveux étant associé à une spécification de couleur de cheveux initiale,
dans lequel le au moins un résultat de coloration comprend une spécification mesurée concernant une propriété de la couleur des cheveux obtenue en appliquant l'agent colorant capillaire aux cheveux ,
diviser (120) les données de couleur de cheveux au moins en un premier groupe de couleurs de cheveux et un deuxième groupe de couleurs de cheveux, le premier groupe de couleurs de cheveux comprenant plus de données que le deuxième groupe de couleurs de cheveux,
ajouter (130) des données de couleur de cheveux artificielles au deuxième groupe de couleurs de cheveux, dans lequel les données de couleur de cheveux artificielles sont générées à l'aide des données de couleur de cheveux obtenues, dans lequel les données de couleur de cheveux artificielles sont ajoutées au deuxième groupe de couleurs de cheveux à l'aide d'une méthode de suréchantillonnage, et
construire (140) un modèle associant des agents colorants capillaires à des résultats de coloration en utilisant une analyse prédictive basée sur les données de couleur de cheveux et incluant les données de couleur de cheveux artificielle,
déterminer, à l'aide du modèle construit, l'agent colorant capillaire associé au résultat de coloration souhaité,
dans lequel l'agent colorant capillaire est un produit de coloration capillaire qui représente la correspondance de couleur la plus proche entre le résultat de coloration souhaité et les résultats de coloration capillaire pouvant être obtenus avec des agents de coloration capillaire existants ou pouvant être fabriqués,
déterminer la recette de l'agent colorant capillaire associé au résultat de coloration souhaité, et
produire la recette.

2. Procédé selon la revendication 1, dans lequel l'analyse prédictive utilise au moins une méthode parmi un groupe de méthodes, dans lequel le groupe de méthodes comprend

la régression linéaire ou multilinéaire,
une régression polynomiale multiple,
une méthode de réseau neuronal,
une méthode de machine à vecteurs de support, et
une méthode d'arbre de décision.

3. Procédé selon l'une quelconque des revendications précédentes, dans lequel la couleur initiale des cheveux est paramétrée dans un espace colorimétrique.

4. Procédé selon l'une quelconque des revendications précédentes, dans lequel des données de couleur de cheveux artificielle sont ajoutées au deuxième groupe de couleurs de cheveux afin d'obtenir un nombre sensiblement identique de points de données dans le premier groupe de couleurs de cheveux et dans le deuxième groupe de couleurs de cheveux.

5. Procédé selon la revendication 1, dans lequel la méthode de suréchantillonnage est basée sur une technique de suréchantillonnage synthétique minoritaire.

6. Procédé selon l'une quelconque des revendications précédentes, dans lequel les données de couleur de cheveux sont divisées en un premier groupe de couleurs de cheveux et un deuxième groupe de couleurs de cheveux sur la base d'une caractéristique liée à la couleur des agents colorants pour cheveux.

7. Procédé selon l'une quelconque des revendications précédentes, dans lequel les données de couleur de cheveux sont divisées en un premier groupe de couleurs de cheveux et un deuxième groupe de couleurs de cheveux sur la base d'une analyse sémantique des propriétés des agents colorants capillaires dans les données de couleur de cheveux.

8. Procédé selon l'une quelconque des revendications précédentes, dans lequel les données de couleur de cheveux sont divisées en un premier groupe de couleurs de cheveux et un deuxième groupe de couleurs de cheveux sur la base d'une évaluation de la composition chimique des agents colorants capillaires parmi la pluralité d'agents colorants capillaires.

9. Procédé selon la revendication 8, dans lequel les agents colorants capillaires comprenant des composés pyrazoliques sont classés dans le deuxième groupe de couleurs de cheveux.

10. Support de stockage non transitoire lisible par ordinateur (210-216) sur lequel est stocké un programme informatique comprenant des instructions pour l'exécution d'un procédé de production d'une recette d'un agent colorant capillaire associé à un résultat de coloration souhaité selon l'une quelconque des revendications précédentes.

100

110

Obtaining hair color data comprising values related to a plurality of hair coloring agents and for each hair coloring agent at least one coloring result

120

Splitting the hair color data at least into a first hair color group and a second hair color group, the first hair color group comprising more data than the second hair color group

130

Adding artificial hair color data to the second hair color group, wherein the artificial hair color data is generated using the obtained hair color data

140

Building a model associating hair coloring agents to coloring results by employing predictive analytics based on the hair color data and including the artificial hair color data

**FIG. 1**

**FIG. 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017103050 A
- WO 2017103056 A
- US 7877294 B2
- WO 2017077498 A1
- WO 2006090363 A1
- JP 2007212140 A
- WO 9641139 A1

### Non-patent literature cited in the description

- **CHAWLA, NITESH ; BOWYER, KEVIN ; HALL, LAWRENCE ; KEGELMEYER, W.** SMOTE: Synthetic Minority Over-sampling Technique. *J. Artif. Intell. Res. (JAIR).*, 2002, vol. 16, 321-357
- **NITESH V. CHAWLA et al.** SMOTE: Synthetic Minority Over-sampling Technique. *Journal of Artificial Intelligence Research*, 2002, vol. 16, 321-357